# EUROPEAN PATENT APPLICATION

(11) **EP 1 870 130 A1**
(43) Date of publication of application: **26.12.2007**
(21) Application number: 06425415.4
(22) Date of filing: 19.06.2006
(51) Int. Cl.: A61N 1/375

(54) **Implantable electrical stimulator with curved housing**

(71) Applicant: Lifestim S.r.l., 35030 Rubano (PD) (IT)
(72) Inventor: Snichelotto, Eugenio, 35030 Rubano (PD) (US)
(74) Representative: Vinci, Marcello

(57) **Abstract**

The invention is a new implantable electrostimulator with curved housing, with a housing formed by two coupled half-shells suitable for containing at least the accumulator and the control and command circuit of the electrostimulator. The two half-shells constituting the body of the new electrostimulator are made of easily deformable biocompatible material, preferably a titanium-based alloy. The two half-shells have a generically arcuate or convex complementary form with raised edges. Said raised edges of the two half-shells are such as to permit coupling of the two half-shells to form a closed housing. Once coupled, the two half-shells enclose both the accumulator and the control and command circuit.

## Description

The present patent concerns electrostimulation devices and in particular refers to implantable electrostimulators.

It is known that analgesic electrostimulation neutralises or in any case alleviates the sensation of pain, especially in cases of chronic pain.

The neuromodulation systems for the treatment of pain are electronic devices which stimulate one or more nerve fibres.

With said stimulation the user experiences a sensation of "touching" instead of a sensation of pain.

In cases of chronic pain, implantable neuromodulation devices, similar to pacemakers, which stimulate one or more nerve fibres, are applied.

The known electrostimulation devices comprise an accumulator, a control and command circuit and one or more electrodes for application of the electrostimulation.

The control and command circuit, powered by the accumulator, generates the electrical impulses whose intensity, potential difference, duration and frequency are set according to different patterns.

Said electrical impulses are applied on the nerve bundles of the patient via the electrodes connected to said control and command circuit.

The control and command circuit and the accumulator are contained in an implantable housing, which constitutes the body of the electrostimulator, to which the electric wire providing the connection to the neurostimulation electrode is connected.

The bodies of the known electrostimulators have a generically parallelepiped shape, the thickness of which can vary, in particular depending on the accumulator used.

All the bodies of the known electrostimulators, also the bodies with reduced thickness, have a substantially flat shape and cannot be implanted in curved parts of the patient's body.

To remedy all the above-mentioned drawbacks, a new implantable electrostimulator with curved housing has been designed and produced.

One object of the new electrostimulator is to permit positioning thereof in points and areas of the patient's body that are not flat.

These and other direct and complementary objects have been achieved through the implementation of the new implantable electrostimulator with curved housing, with a housing formed by two coupled half-shells suitable for containing at least the accumulator and the control and command circuit of the electrostimulator.

The two half-shells constituting the body of the new electrostimulator are made of easily deformable biocompatible material, preferably a titanium-based alloy.

The two half-shells have a complementary shape, generically arcuate or convex with raised edges. Said raised edges of the two half-shells are such as to permit coupling of the two half-shells to form a closed housing.

The two half-shells, once coupled, enclose both the accumulator and the control and command circuit.

During production the two half-shells are appropriately formed, according to a number of predefined forms and curvatures which can be used in the majority of cases. The accumulator, the control and command circuit and any other parts are appropriately positioned between the half-shells. Lastly, said two coupled half-shells enclosing the various parts are closed and sealed.

If necessary, the free spaces between the accumulator, the control and command circuit and the other parts are filled with inert lightweight filler material.

After closing, the body of the electrostimulator and the two half-shells that make it up are non-deformable.

The body of the electrostimulator thus formed has exactly the right shape to be applied and implanted in the required position.

The characteristics of the new implantable electrostimulator with curved housing will be highlighted in greater detail in the following description with reference to the drawings, attached as non-limiting examples.

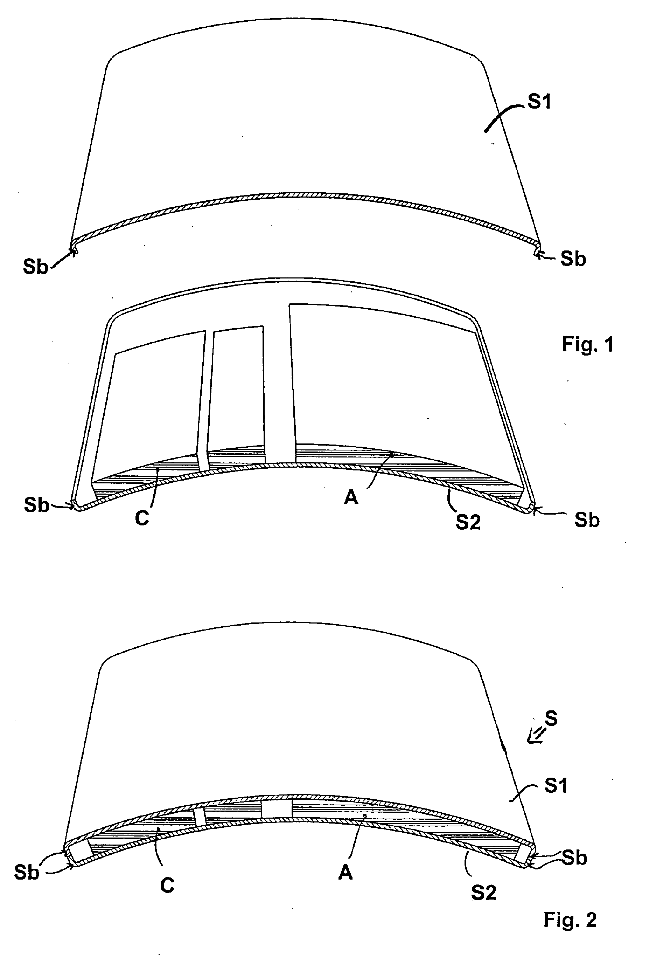

Figure 1 shows a section of the new electrostimulator open, while Figure 2 shows a section of the new electrostimulator closed.

The new implantable electrostimulator with curved housing comprises a housing (S) formed by two half-shells (S1, dS2) which can be coupled and are suitable for containing at least the accumulator (A) and the control and command circuit (C) of the electrostimulator.

The two half-shells (S1, S2) making up the body (S) of the new electrostimulator are shaped in a complementary manner and made of easily deformable biocompatible material, preferably a titanium-based alloy.

The two half-shells (S1, S2), once coupled, enclose both the accumulator (A) and the control and command circuit (C).

The two half-shells (S1, S2) have a complementary shape, generically arcuate or convex with raised edges (Sb). Said raised edges (Sb) of the two half-shells (S1, S2) are such as to permit coupling of the two half-shells (S1, S2), so that said two half-shells (S1, S2) when coupled form a closed housing (S).

During production the two half-shells (S1, S2) are appropriately shaped, according to a number of predefined forms and curvatures which can be used in the majority of cases. The accumulator (A), the control and command circuit (C) and any other parts are appropriately positioned between the half-shells (S1, S2). Lastly, said two half-shells (S1, S2), coupled and enclosing the various parts, are closed and sealed.

It is possible to provide for the empty spaces between the accumulator (A), the control and command circuit (C) and the other parts to be filled with inert lightweight filler material.

After closing, the body (S) of the electrostimulator and the two half-shells (S1, S2) that make it up are non-deformable.

The body (S) of the electrostimulator thus formed has exactly the right shape to be applied and implanted in the required position.

The new implantable electrostimulator with curved housing (S) has considerable advantages.

The new electrostimulator can be positioned in points and areas of the patient's body that are not flat.

The new electrostimulator can be positioned near the neurostimulation point, so that the implant operation is not particularly invasive.

## Claims

1. Implantable electrostimulator, comprising a housing (S) made of biocompatible material suitable for containing at least one accumulator (A) and one control and command circuit (C), **characterised in that** said housing (S) consists of at least two complementary coupled half-shells (S1, S2), and wherein said half-shells (S1, S2) are not flat.

2. Implantable electrostimulator according to claim 1, **characterised in that** the two complementary half-shells (S1, S2) are arcuate.

3. Implantable electrostimulator according to claim 1, **characterised in that** the two complementary half-shells (S1, S2) are convex.

4. Implantable electrostimulator according to the preceding claims, **characterised in that** said half-shells (S1, S2) have raised edges (Sb), and wherein said raised edges (Sb) of the two half-shells (S1, S2) are such as to permit coupling of the two half-shells (S1, S2) to form a closed housing (S).

5. Implantable electrostimulator according to the preceding claims, **characterised in that** it uses inert lightweight filler material to fill the spaces inside the housing (S) between the accumulator (A), the control and command circuit (C) and any other parts contained in the housing (S).
